# EUROPEAN PATENT APPLICATION

(11) **EP 4 083 054 A1**
(43) Date of publication of application: **02.11.2022**
(21) Application number: 20907668.6
(22) Date of filing: 22.12.2020
(51) Int. Cl.: C07K 14/00, C07K 1/06

(54) **METHOD FOR PRODUCING PNA OLIGOMER IN SOLUTION PROCESS**

(30) Priority: 24.12.2019 KR 20190174492
(71) Applicant: Seasunbio Materials, Daejeon 34015 (KR)
(72) Inventor: PARK, Hee Kyung, Daejeon 34034 (KR); KIM, Yongtae, Sejong-si 30063 (KR); JEON, Juhyeon, Daejeon 34522 (KR); JUNG, Jinwoo, Daejeon 34308 (KR); HONG, In Seok, Sejong-si 30100 (KR)
(74) Representative: Stolmár & Partner Patentanwälte PartG mbB
(86) International application number: PCT/KR2020/018912
(87) International publication number: WO 2021/133033

(57) **Abstract**

The present invention provides a method for producing a PNA oligomer. More specifically, the method comprises a step for reacting a PNA block and a PNA block in a solution to produce a PNA oligomer, and thus a PNA oligomer of interest can be produced with remarkably improved purity and yield.

## Description

### TECHNICAL FIELD

The present invention relates to a method for producing a PNA oligomer in a solution process.

### BACKGROUND ART

A nucleic acid is DNA and RNA responsible for genetic information of living things, as is known.

On the other hand, a peptide nucleic acid (PNA) is a nucleic acid in which a sugar phosphate backbone of a nucleic acid is converted into a N-(2-aminoethyl)glycine backbone.

Since the sugar phosphate backbone of DNA/RNA is negatively charged under neutral conditions, there is electrostatic repulsion between complementary chains, but since the backbone structure of PNA originally has no charge, there is no electrostatic repulsion.

That is, a PNA main chain has substantially no charge, and this is a very important characteristic of PNA, and due to this characteristic, PNA may be used for various purposes for which it is difficult to use a natural oligonucleotide or an oligonucleotide derivative.

Furthermore, since PNA binds to DNA or RNA with higher affinity than a natural oligonucleotide, it is very stable in a serum as compared with natural DNA.

However, though the interest and study on the utilization of PNA are diversifying and increasing, the study on a synthesis method of a PNA oligomer is in a sluggish state.

Usually, since a synthesis method of PNA oligomer uses synthesis of a solid-phase peptide, when a PNA monomer unit is classified by the backbone structure of PNA, it may be classified into two types, a Fmoc type PNA monomer unit and a Boc type PNA monomer unit.

The synthesis method of the Fmoc type PNA monomer unit has been already established, but its mass production is not easy, and low yield and purity are problematic.

As an example, a monomer which may easily synthesize PNA with a high yield is known in WO2005-009998 A1, but it is not economical for mass production due to a plurality of processes.

Therefore, a method for producing a PNA oligomer in a solution process, which may efficiently produce desired PNA with high purity and yield by a simple process, is needed.

### DISCLOSURE

### TECHNICAL PROBLEM

An object of the present invention is to provide a method for producing a desired PNA oligomer with surprisingly improved purity and yield by a simple solution process.

### TECHNICAL SOLUTION

In one general aspect, a method for producing a PNA oligomer with extremely improved purity and yield in a simple and easy solution process is provided, and the method for producing a PNA oligomer includes:
reacting a PNA block with a PNA block in a solution phase to produce a PNA oligomer,
wherein the PNA block is two or more identical or different PNA monomers bound to each other.

Preferably, the method for producing a PNA oligomer of the present invention may include:
in the presence of a coupling reagent or a solvent,
mixing a first PNA dimer represented by the following Chemical Formula 1, a first PNA trimer represented by the following Chemical Formula 2, or a first PNA tetramer represented by the following Chemical Formula 3 with a second PNA dimer represented by the following Chemical Formula 4, a second PNA trimer represented by the following Chemical Formula 5, or a second PNA tetramer represented by the following Chemical Formula 6, and then adding an amine compound to produce a PNA oligomer represented by the following Chemical Formulae 7 to 9: 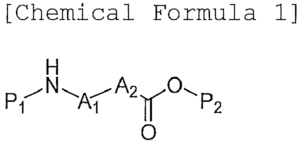 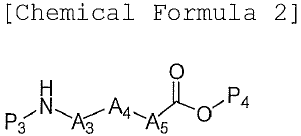 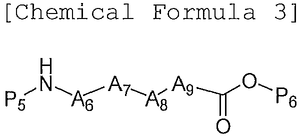 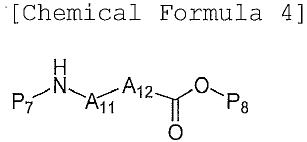 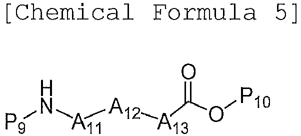 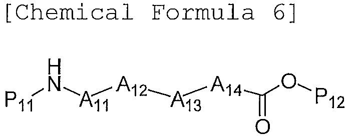 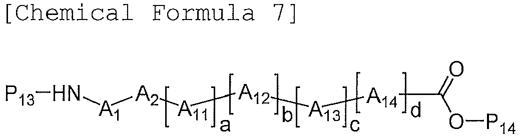 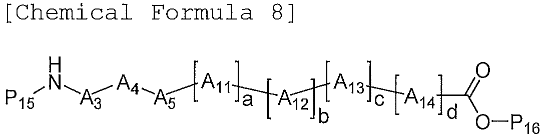 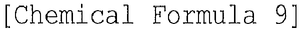 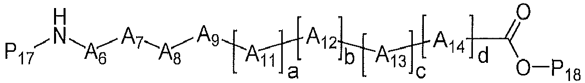 wherein
   A₁ to A₉ and A₁₁ to A₁₄ are independently of one another PNA monomers including a nucleic acid base, which are identical to or different from each other;
   P₁ to P₁₈ are independently of one another hydrogen or protecting groups identical to or different from each other, but each of P₁ and P₂, P₃ and P₄, P₅ and P₆, P₁₁ and P₁₂, P₁₃ and P₁₄, P₁₅ and P₁₆, and P₁₇ and P₁₈ is not hydrogen at the same time; and
   a and b are independently of each other an integer of 1, and c and d are independently of each other an integer of 0 or 1.

Preferably, the method for producing a PNA oligomer according to an exemplary embodiment of the present invention may include:
a) in the presence of a coupling reagent or a solvent,
   adding a second PNA dimer represented by the following Chemical Formula 4-1, a second PNA trimer represented by the following Chemical Formula 5-1, or a second PNA tetramer represented by the following Chemical Formula 6-1 to a first PNA dimer represented by the following Chemical Formula 1-1, a first PNA trimer represented by the following Chemical Formula 2-1, or a first PNA tetramer represented by the following Chemical Formula 3-1 to obtain a mixed solution; and [0044] b) adding an amine compound to the mixed solution of a) to produce the compounds of Chemical Formulae 7 and 8: 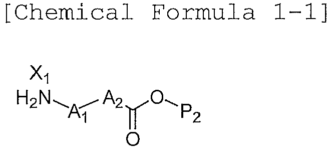 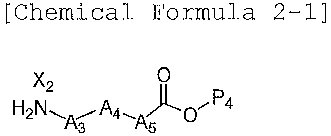 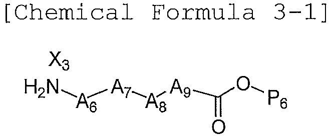 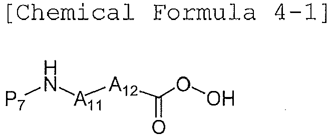 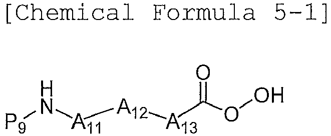 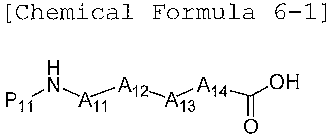 wherein
   A₁ to A₉ and A₁₁ to A₁₄ are independently of one another PNA monomers including a nucleic acid base, which are identical to or different from each other;
   P₂, P₄, P₆, P₇, P₉, and P₁₁ are independently of one another hydrogen or protecting groups identical to or different from each other;
   X₁ to X₃ are independently of one another an acid salt; and
   a and b are independently of each other an integer of 1, and c and d are independently of each other an integer of 0 or 1.

Preferably, the amine compound according to an exemplary embodiment of the present invention is not limited, but may be N,N-diisopropylethylamine.

Preferably, the method for producing a PNA oligomer according to an exemplary embodiment of the present invention may further include: using a product from step b) as a starting material again to repeat steps a) and b).

More preferably, a volume ratio of the starting material and the solvent in the mixed solution may be 1:10 or more.

The nucleic acid base according to an exemplary embodiment of the present invention may be adenine, cytosine, 5-methylcytosine, guanine, thymine, uracil, purine, 2,6-diaminopurine, N⁴N⁴-ethanocytosine, N⁶N⁶-ethano-2,6-diaminopurine, 5-(C3-C6)-alkynyluracil, 5-(C3-C6)-alkynylcytosine, 5-(1-propargylamino)uracil, 5-(1-propargylamino)cytosine, phenoxazine, 9-aminoethoxyphenoxazine, 5-fluorouracil, pseudoisocytosine, 5-(hydroxymethyl)uracil, 5-aminouracil, pseudouracil, dihydrouracil, 5-(C1-C6)-alkyluracil, 5-(C1-C6)-alkylcytosine, 5-(C2-C6)-alkenylcytosine, 5-fluorocytosine, 5-chlorouracil, 5-chlorocytosine, 5-bromouracil, 5-bromocytosine, 7-deazaadenine, 7-deazaguanine, 8-azapurine, 7-deaza-7-substituted purine, thiouracil, or an artificial nucleic acid base.

The protecting group according to an exemplary embodiment of the present invention may be fluorenylmethoxycarbonyl, tert-butyloxycarbonyl, benzyloxycarbonyl, benzhydryloxycarbonyl (Bhoc), acetyl, benzoyl, benzyl, carbamate, p-methoxybenzyl, 3,4-dimethoxybenzyl, p-methoxyphenyl, tosyl, trichloroethyl chloroformate, sulfonamides, or isobutyryl.

Preferably, the first PNA dimer or the second PNA dimer according to an exemplary embodiment of the present invention may be represented by the following Chemical Formula 11,
the first PNA trimer or the second PNA trimer may be represented by the following Chemical Formula 12, and
the first PNA tetramer or the second PNA tetramer may be represented by the following Chemical Formula 13: 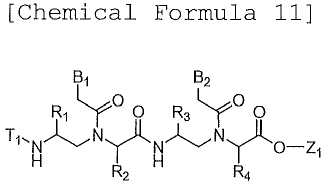 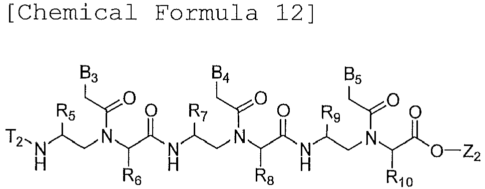 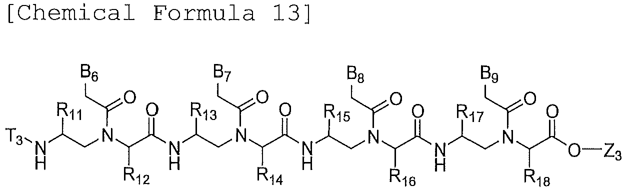 wherein
   R₁ to R₁₈ are independently of one another hydrogen or an amino acid residue having a residue or substituent of an amino acid;
   T₁ to T₃ are independently of one another an amine protecting group;
   Z₁ to Z₃ are independently of one another a hydroxy protecting group; and
   B₁ to B₉ are independently of one another a nucleic acid base with or without an amine protecting group.

The first PNA dimer represented by Chemical Formula 4-1, the first PNA trimer represented by Chemical Formula 5-1, or the first PNA tetramer represented by Chemical Formula 6-1 may be used at 1:0.8 to 1.2 mol with respect to 1 equivalent of the first PNA dimer represented by Chemical Formula 1-1, the first PNA trimer represented by Chemical Formula 2-1, or the first PNA tetramer represented by Chemical Formula 3-1.

Preferably, the coupling reagent according to an exemplary embodiment of the present invention may be HBTU, PyBop, or a mixture thereof, and step b) may be performed at -10 to 5°C for 10 to 60 minutes.

The solvent according to an exemplary embodiment of the present invention may be a mixed solvent of chlorinated (C1-C4)alkane, DMF, and DIEA.

A step of adding water to the compound of Chemical Formulae 7 to 9 according to an exemplary embodiment of the present invention and performing separation and purification may be further included.

### ADVANTAGEOUS EFFECTS

The method for producing a PNA oligomer of the present invention includes producing a PNA block and reacting the PNA blocks with each other to produce a PNA oligomer, thereby significantly decreasing PNA production steps, and thus, the PNA oligomer may be produced with a high purity in a very economical manner.

Specifically, in the method for producing a PNA oligomer of the present invention, the PNA oligomer is produced using a PNA dimer, a PNA trimer, or a PNA tetramer, and thus, the PNA dimer may be produced by a simpler process than a conventional method using a PNA monomer, and a desired PNA oligomer may be produced more accurately.

Therefore, in the method for producing a PNA oligomer of the present invention, the PNA oligomer is produced by fewer process steps than the conventional method using a PNA monomer, and separation from by-products is very easy, and thus, the yield and the purity of the PNA oligomer produced are extremely high.

Furthermore, in the method for producing a PNA oligomer, since all processes may be performed in a solution phase, mass production is allowed, and the method is very favorable to commercialization.

In addition, the method for producing a PNA oligomer of the present invention is performed using a PNA block, specifically, a PNA dimer, a PNA trimer, or a PNA tetramer, thereby using very small amounts of PNA dimer, PNA trimer, or PNA tetramer as compared with the conventional method, and thus, the method is very economical and allows easy mass production of the PNA oligomer with high yield and purity.

### DESCRIPTION OF DRAWINGS

FIG. 1 is results of HPLC measurement of a PNA oligomer produced in Example 20 of the present invention.
FIG. 2 is results of HPLC measurement of a PNA oligomer produced in Comparative Example 1 of the present invention.

### BEST MODE

Hereinafter, the method for producing a PNA oligomer of the present invention and the PNA oligomer produced therefrom will be described in detail; however, technical terms and scientific terms used herein have the general meaning understood by those skilled in the art to which the present invention pertains unless otherwise defined, and a description for the known function and configuration obscuring the present invention will be omitted in the following description.

An amino acid described in the present specification is used in a broadest sense, and includes not only natural amino acids, for example, serine (Ser), asparagine (Asn), valine (Val), leucine (Leu), isoleucine (Ile), alanine (Ala), tyrosine (Tyr), glycine (Gly), lysine (Lys), arginine (Arg), histidine (His), aspartic acid (Asp), glutamic acid (Glu), glutamine (Gln), threonine (Thr), cysteine (Cys), methionine (Met), phenylalanine (Phe), tryptophane (Trp), and proline (Pro), but also unnatural amino acids such as amino acid variants and derivatives. A person skilled in the art will understand that the amino acid in the present specification may include, for example, chemically modified amino acids such as L-amino acid; D-amino acid; and amino acid variants and derivatives; amino acids which are not an in-vivo protein forming constituent material such as norleucine, β-alanine, and ornithine; chemically synthesized compounds having amino acid properties known to a person skilled in the art, and the like, considering the broad definition as such. An example of the unnatural amino acid may include α-methyl amino acid (such as α-methyl alanine), D-amino acid, histidine-like amino acid (such as 2-amino-histidine, β-hydroxy-histidine, homohistidine, α-fluoromethyl-histidine, and α-methyl-histidine), amino acid having extra methylene in a side chain ("homo" amino acid), and amino acid in which a carboxyl acid functional group amino acid in a side chain is substituted by a sulfonic acid group (such as cysteic acid), in addition to a threonine derivative A.

An "amino acid residue having a substituent" described in the present specification means that an amino acid residue has a substituent, may include, as an example, alanine, cysteine, aspartic acid, glutamic acid, phenylalanine, glycine, histidine, isoleucine, lysine, leucine, methionine, asparagine, proline, glutamine, arginine, serine, valine, tryptophan, or tyrosine, which is substituted by an acetyl group, and may be a peptide bound to an amino acid.

A "protecting group" described in the present invention is a functional group for protecting a specific functional group, as an example, an amine group, a hydroxyl group, and the like in an organic reaction and may be any functional group as long as it is in a range which may be recognized by a person skilled in the art of organic synthesis, and a specific example of an amine protecting group may include fluorenylmethoxycarbonyl (Fmoc), tert-butyloxycarbonyl (Boc), benzyloxycarbonyl (Cbz), Bhoc, allyloxycarbonyl (Alloc), acetyl, benzoyl, benzyl, carbamate, p-methoxybenzyl, 3,4-dimethoxybenzyl, p-methoxyphenyl, tosyl, trichloroethyl chloroformate, sulfonamides (Bts, Nosyl&Nps), and isobutyryl.

"Chlorinated alkane" described in the present invention means that one or more hydrogens of alkane are substituted by chlorine, in which alkane includes both linear and branched types, and alkane has 1 to 10 carbon atoms, preferably 1 to 7 carbon atoms, and more preferably 1 to 4 carbon atoms, unless otherwise particularly stated.

Regarding the expressions "substituted" and "having substituent" and a substituent described in the present specification, unless otherwise stated, an optionally substituted substituent of the present invention may be halogen, hydroxyl, carboxyl acid group, nitro, cyano, (lower) alkyl, haloalkyl, mono- or di-alkylamino, alkoxy, thioalkyl, cycloalkyl, heterocycloalkyl, aryl, heteroaryl, -NO₂, -NRₐ₁R_{b1}, -NRₐ₁C(=O) R_{b1}, -NRₐ₁C(=O)NRₐ₁R_{b1}, -NRₐ₁C(=O)OR_{b1}, -NRₐ₁SO₂R_{b1}, -ORₐ₁, -CN, -C(=O)Rₐ₁, -C(=O)ORₐ₁, -C(=O)NRₐ₁R_{b1}, - OC(=O)Rₐ₁, -OC(=O)ORₐ₁, -OC(=O)NRₐ₁R_{b1}, -NRₐ₁SO₂R_{b1}, -PO₃Rₐ₁, - PO(ORₐ₁)(OR_{b1}), -SO₂Rₐ₁, -S(O)Rₐ₁, -SO(NRₐ₁)R_{b1} (e.g., sulfoximine), -S(NRₐ₁)R_{b1} (e.g., sulfilimine), and -SRₐ₁, wherein Rₐ₁ and R_{b1} are the same as or different from each other, and may be independently of each other hydrogen, halogen, amino, alkyl, alkoxyalkyl, haloalkyl, aryl, or heterocycle, or Rₐ₁ and R_{b1} may form a heterocycle with a nitrogen atom attached thereto. Here, the number of Rₐ₁ and R_{b1} may be plural depending on the atom attached thereto, and preferably, alkyl may be C₁₋₆ alkyl, cycloalkyl and heterocycloalkyl may be C₃₋₁₂, aryl may be C₆₋₁₂, and heterocycle and heteroaryl may be C₃₋₁₂.

A PNA monomer described in the present invention means a nucleic acid base with or without an amine or hydroxyl protecting group at a N position of a PNA basic backbone, specifically, N-(2-aminoethyl)glycine (Compound 5) or Compound 8.

Specifically, the following structures such as those having no hydroxyl group or no amine group are all included:

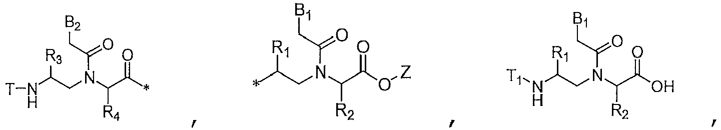

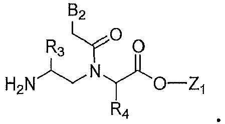

A PNA dimer described in the present invention is two PNA monomers bound to a nucleic acid base at a N position of N-(2-aminoethyl)glycine (Compound 5) or Compound 8, which is a PNA basic backbone, linked to each other, and the nucleic acid bases included in the PNA dimer may be the same as or different from each other, and specifically may be represented by Chemical Formula 11.

A PNA trimer described in the present invention is three PNA monomers bound to a nucleic acid base at a N position of N-(2-aminoethyl)glycine (Compound 5) or Compound 8, which is a PNA basic backbone, linked to each other, and the nucleic acid bases included in the PNA trimer may be the same as or different from each other, and specifically may be represented by Chemical Formula 12.

A PNA tetramer described in the present invention also means a structure in which four PNA monomers are linked like the PNA trimer, and specifically, may be represented by Chemical Formula 13.

A "PNN block" described in the present specification means that two or more PNA monomers are linked to each other, and may refer to a PNA dimer, a PNA trimer, a PNA tetramer, and the like.

The present invention provides a method for producing a PNA oligomer with high purity and yield in a solution process, and the method for producing a PNA oligomer includes:
reacting a PNA block with a PNA block in a solution phase to produce a PNA oligomer,
wherein the PNA block is two or more identical or different PNA monomers bound to each other.

In the method for producing a PNA oligomer of the present invention, unlike a conventional method for producing a PNA oligomer by connecting each PNA monomer to a support, a PNA block in which two or more PNA monomers are linked in a solution phase is produced, and the PNA blocks are reacted to produce a PNA oligomer, thereby producing a desired PNA oligomer more accurately by fewer process steps.

Furthermore, in the method for producing a PNA oligomer of the present invention, a PNA block is used to allow production of a PNA oligomer with higher purity and yield, and all reactions proceed in a solution to allow easier production and mass production.

Preferably, the method for producing a PNA oligomer of the present invention may include:

in the presence of a coupling reagent or a solvent, mixing a first PNA dimer represented by the following Chemical Formula 1, a first PNA trimer represented by the following Chemical Formula 2, or a first PNA tetramer represented by the following Chemical Formula 3 with a second PNA dimer represented by the following Chemical Formula 4, a second PNA trimer represented by the following Chemical Formula 5, or a second PNA tetramer represented by the following Chemical Formula 6, and then adding an amine compound to produce a PNA oligomer represented by the following Chemical Formulae 7 to 9: 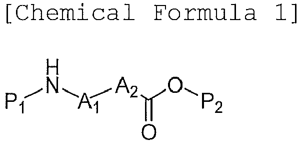 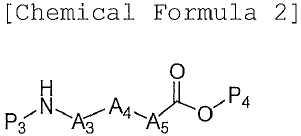 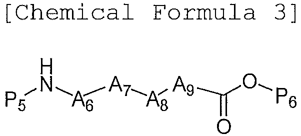 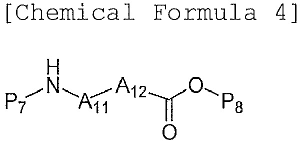 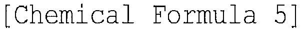 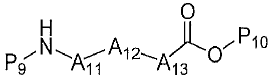 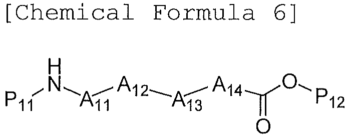 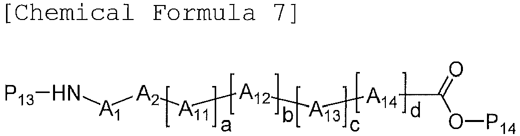 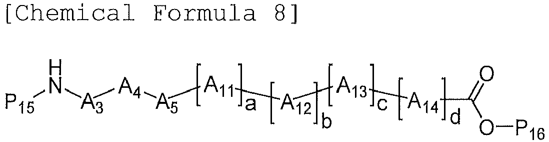 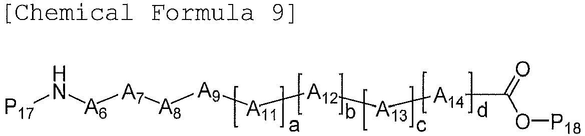 wherein
A₁ to A₉ and A₁₁ to A₁₄ are independently of one another PNA monomers including a nucleic acid base, which are identical to or different from each other;
P₁ to P₁₈ are independently of one another hydrogen or protecting groups identical to or different from each other, but each of P₁ and P₂, P₃ and P₄, P₅ and P₆, P₁₁ and P₁₂, P₁₃ and P₁₄, P₁₅ and P₁₆, and P₁₇ and P₁₈ is not hydrogen at the same time; and
a and b are independently of each other an integer of 1, and c and d are independently of each other an integer of 0 or 1.

Unlike a conventional method for producing a PNA oligomer by binding a PNA monomer to a support (resin), which is then washed, and binding a PNA monomer thereto again, the method for producing a PNA oligomer according to an exemplary embodiment of the present invention uses a PNA dimer, a PNA trimer, and a PNA tetramer which are PNA blocks, and thus, is very economical due to reduced synthesis steps, and may produce the PNA oligomer with higher yield and purity than the production method in a solid phase.

Furthermore, since the PNA oligomer is produced using the PNA dimer, the PNA trimer, or the PNA tetramer of the present invention, process steps are reduced to make the method economical, an amine compound which is a specific compound in a solution phase is added to a mixture of a starting material and a coupling reagent, so that by-products are fewer produced to allow production of the PNA oligomer with high yield and purity.

That is, unlike a method of mixing a starting material, a coupling reagent, and an amine compound to produce a PNA oligomer, in the method for producing a PNA oligomer of the present invention, an amine compound is added to a mixed solution of a starting material and a coupling reagent, thereby significantly decreasing production of by-products to produce the PNA oligomer with high yield and purity.

Besides, it is very easy to separate and purify the PNA oligomer produced therefrom.

Specifically, the method for producing a PNA oligomer according to an exemplary embodiment uses a PNA dimer, a PNA trimer, or a PNA tetramer which may be synthesized by a solution process, thereby not producing a PNA oligomer having n-1 nucleic acid bases or a PNA oligomer having n-2 nucleic acid bases as by-products, in the production of a PNA oligomer having n nucleic acid bases, and thus, it is very easy to separate the PNA oligomer from impurities such as other by-products, and the purity of the separated PNA oligomer is very high.

More Preferably, the method for producing a PNA oligomer according to an exemplary embodiment of the present invention may include:
a) in the presence of a coupling reagent or a solvent,
   adding a second PNA dimer represented by the following Chemical Formula 4-1, a second PNA trimer represented by the following Chemical Formula 5-1, or a second PNA tetramer represented by the following Chemical Formula 6-1 to a first PNA dimer represented by the following Chemical Formula 1-1, a first PNA trimer represented by the following Chemical Formula 2-1, or a first PNA tetramer represented by the following Chemical Formula 3-1 to obtain a mixed solution; and
b) adding an amine compound to the mixed solution of a) to produce the compounds of Chemical Formulae 7 to 9: 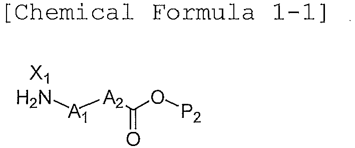 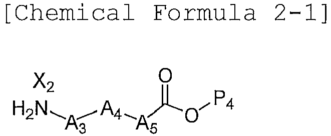 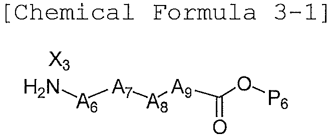 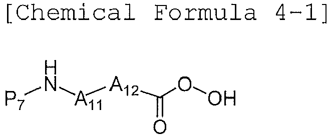 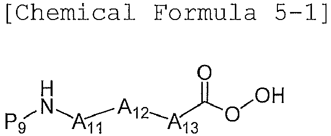 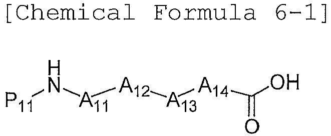 wherein
   A₁ to A₉ and A₁₁ to A₁₄ are independently of one another PNA monomers including a nucleic acid base, which are identical to or different from each other;
   P₂, P₄, P₆, P₇, P₉, and P₁₁ are independently of one another hydrogen or protecting groups identical to or different from each other;
   X₁ to X₃ are independently of one another an acid salt; and
   a and b are independently of each other an integer of 1, and c and d are independently of each other an integer of 0 or 1.

Preferably, N,N-diisopropylethylamine which is a specific amine compound is used in terms of converting the amine salts of Chemical Formulae 1-1, 2-1, and 3-1 into free amines while also increasing reactivity with hydroxy of Chemical Formulae 4-1, 5-1, and 6-1, thereby producing the PNA oligomer with more improved yield and purity.

Preferably, the amine compound according to an exemplary embodiment of the present invention may be used at 5 to 40 mol, more preferably 10 to 30 mol, with respect to 1 mol of the compounds of Chemical Formulae 1-1, 2-1, and 3-1.

In addition, the method for producing a PNA oligomer according to an exemplary embodiment of the present invention may further include: using a product from step b) as a starting material again to repeat steps a) and b).

The method for producing a PNA oligomer according to an exemplary embodiment of the present invention uses a PNA dimer, a PNA trimer, or a PNA tetramer, so that a capping step for protecting an unreacted functional group is not needed, unlike a conventional method, and thus, production steps may be significantly reduced to allow mass production.

The PNA oligomer according to an exemplary embodiment of the present invention may easily produce the desired number of nucleic acid bases, and may include, preferably 4 or more nucleic acid bases, and more preferably 4 to 30 nucleic acid bases.

A volume ratio of the starting material and the solvent in the mixed solution according to an exemplary embodiment of the present invention may be 1:10 or more, preferably 1:10 to 250, and more preferably 1:10 to 100.

When the volume ratio of the starting material and the solvent in the mixed solution according to an exemplary embodiment of the present invention is 1:10 or more, the PNA oligomer may be produced with higher yield and purity.

The nucleic acid base according to an exemplary embodiment of the present invention may be adenine, cytosine, 5-methylcytosine, guanine, thymine, uracil, purine, 2,6-diaminopurine, N⁴N⁴-ethanocytosine, N⁶N⁶-ethano-2,6-diaminopurine, 5-(C3-C6)-alkynyluracil, 5-(C3-C6)-alkynylcytosine, 5-(1-propargylamino)uracil, 5-(1-propargylamino)cytosine, phenoxazine, 9-aminoethoxyphenoxazine, 5-fluorouracil, pseudoisocytosine, 5-(hydroxymethyl)uracil, 5-aminouracil, pseudouracil, dihydrouracil, 5-(C1-C6)-alkyluracil, 5-(C1-C6)-alkylcytosine, 5-(C2-C6)-alkenylcytosine, 5-fluorocytosine, 5-chlorouracil, 5-chlorocytosine, 5-bromouracil, 5-bromocytosine, 7-deazaadenine, 7-deazaguanine, 8-azapurine, 7-deaza-7-substituted purine, thiouracil, or an artificial nucleic acid base.

The protecting group according to an exemplary embodiment of the present invention may be a fluorenylmethoxycarbonyl, tert-butyloxycarbonyl, benzyloxycarbonyl, benzhydryloxycarbonyl (Bhoc), acetyl, benzoyl, benzyl, carbamate, p-methoxybenzyl, 3,4-dimethoxybenzyl, p-methoxyphenyl, tosyl, trichloroethyl chloroformate, sulfonamides, or isobutyryl, and preferably, an amine protecting group may be tert-butyloxycarbonyl, benzyloxycarbonyl, benzhydryloxycarbonyl (Bhoc), acetyl, benzoyl, or benzyl, and a protecting group of a hydroxyl group may be (C1-C4)alkyl.

Specifically, the first PNA dimer or the second PNA dimer according to an exemplary embodiment of the present invention may be represented by the following Chemical Formula 11,
the first PNA trimer or the second PNA trimer may be represented by the following Chemical Formula 12, and
the first PNA tetramer or the second PNA tetramer may be represented by the following Chemical Formula 13: 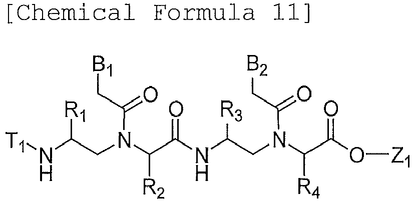 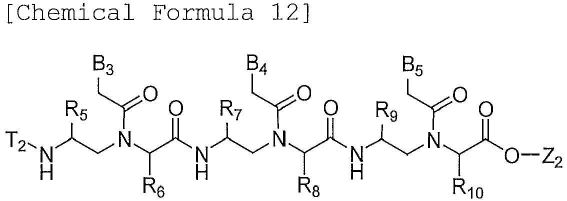 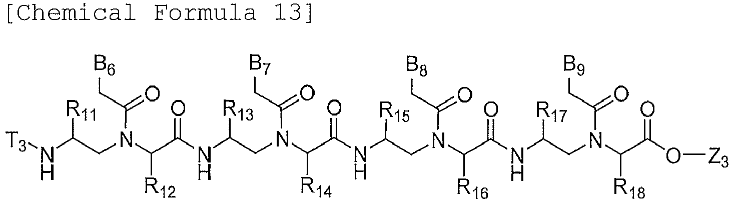 wherein
   R₁ to R₁₈ are independently of one another hydrogen or an amino acid residue having a residue or substituent of an amino acid;
   T₁ to T₃ are independently of one another an amine protecting group;
   Z₁ to Z₃ are independently of one another a hydroxy protecting group; and
   B₁ to B₉ are independently of one another a nucleic acid base with or without an amine protecting group.

The first PNA dimer represented by Chemical Formula 4-1, the first PNA trimer represented by Chemical Formula 5-1, or the first PNA tetramer represented by Chemical Formula 6-1 may be used at 1:0.8 to 1.2 mol, preferably 1:0.9 to 1.1 mol with respect to 1 mol of the first PNA dimer represented by Chemical Formula 1-1, the first PNA trimer represented by Chemical Formula 2-1, or the first PNA tetramer represented by Chemical Formula 3-1 according to an exemplary embodiment of the present invention.

The coupling reagent according to an exemplary embodiment of the present invention is not limited, but may be HBTU, (benzotriazol-1-yloxy)tripyrrolidinophosphonium hexafluorophosphate (PyBop), or a mixture thereof, preferably *N,N,N',N'*-Tetramethyl-*O*-(1*H*-benzotriazol-1-yl)uronium hexafluorophosphate (HBTU).

The amount of the coupling reagent used is also not limited, but may be used at 1.2 to 2.5 mol, preferably 1.2 to 2.0 mol, with respect to 1 mol of the first PNA dimer represented by Chemical Formula 1-1, the first PNA trimer represented by Chemical Formula 2-1, or the first PNA tetramer represented by Chemical Formula 3-1.

Step b) according to an exemplary embodiment of the present invention may be performing a coupling reaction, at -10 to 5°C for 10 to 60 minutes, more preferably at -5 to 5°C for 10 to 40 minutes.

The solvent according to an exemplary embodiment of the present invention may be a mixed solvent of chlorinated (C1-C4)alkane, DMF, and DIEA, and preferably DMF.

Here, the solvent to be used may be a mixed solvent of chlorinated (C1-C4)alkane, dimethylformamide (DMF), and N,N-diisopropylethylamine (DIEA), and the chlorinated (C1-C4)alkane may be one or two or more selected from trichloromethane, dichloromethane, chloromethane, 1,1,2-trichloroethane, 1,1,1-trichloroethane, 1,2-dichloroethane, 1,1-dichloroethane, and chloroethane.

By using DMF, DIEA, or chlorinated alkane as the solvent according to an exemplary embodiment of the present invention, the solubility of a reactant in the coupling reaction is appropriately adjusted to obtain the product with high yield and purity, and in this respect, it may be more preferably DMF.

The PNA oligomer of Chemical Formulae 7 to 9 produced according to an exemplary embodiment of the present invention may be separated and purified by various methods which may be recognized by a person skilled in the art, but preferably, water is added to perform separation and purification.

In addition, the entire processes of the method for producing a PNA oligomer according to an exemplary embodiment of the present invention are performed in a solution phase, and the PNA dimer, the PNA trimer, or the PNA tetramer is used, thereby producing the PNA oligomer with surprisingly improved yield and purity as compared with a conventional method for producing a PNA oligomer from a PNA monomer in a solid phase using a support (resin), and thus, it is a very efficient and economical method.

That is, the method for producing a PNA oligomer of the present invention allows production of all of the first PNA dimer, the first PNA trimer, the first PNA tetramer, the second PNA dimer, the second PNA trimer, the second PNA tetramer, and the PNA oligomer in a solution phase, thereby producing the PNA oligomer having excellent purity and yield by a simple process to allow mass production.

As a result of high performance liquid chromatography (HPLC) analysis, the PNA oligomer according to an exemplary embodiment of the present invention may have a purity of 70% or more, preferably 75% or more, and more preferably 80% or more.

### (Developing solvent: TFA 0.1% in water, TFA 0.1% in MeCN, gradient condition UV detector 260 nm, column 250 mm*4.6 mm)

In addition, the method for producing a PNA oligomer of the present invention performs synthesis without a capping process, and as a result of high performance liquid chromatography (HPLC) analysis under the conditions, the produced PNA oligomer provides a PNA oligomer having a purity of 70% or more, preferably 75% or more, and more preferably 80% or more.

Hereinafter, the present invention will be described in detail by the following Examples, however, the scope of the present invention is not limited thereto.

The organic solvent used in the reaction was purchased from Novabiochem, Alfa aesar, SAMCHUN CHEMICALS, Junsei chemicals co., Ltd., DUKSAN reagents chemical, and the like, and was used without separate additional purification. ¹H-NMR analysis of the synthesized compound was performed at room temperature using Bruker 400 or 500 Mhz, and as a HPLC (waters 1525 Binary hplc pump) developing solvent, a solvent having a ratio of MeCN containing 0.1% of TFA : water containing 0.1% of TFA of 5:95 was used, the ratio of the developing solvent was gradually changed so that a ratio of MeCN containing 0.1% of TFA : water containing 0.1% of TFA was changed to 20:80 for 20 minutes, and then for 10 minutes, a solvent having a ratio of MeCN containing 0.1% of TFA : water containing 0.1% of TFA of 95:5 was used, thereby performing analysis by a column heater at 60°C.

The nucleic acid bases or the nucleic acid bases having an amine protecting group of the following Compounds 1 to 4 were produced in the same manner as in Korean Patent Registration No. 10-0464261.

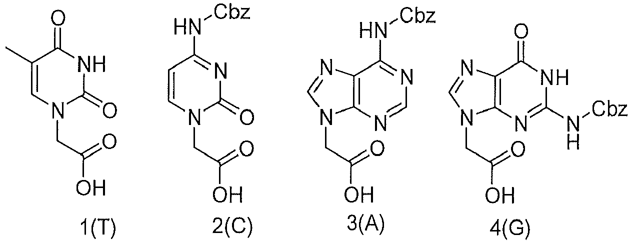

### [Preparation Example 1] Preparation of Compound 5 (Boc-aeg-OEt)

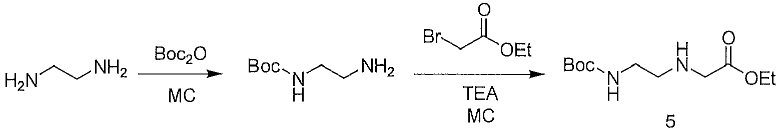

11.0 g of ethylenediamine (183 mmol) was added to a 500 mL of an Erlenmeyer flask, and dissolved in methylene chloride (MC). 5.0 g of Boc₂O (22.9 mmol) was dissolved in MC and added dropwise thereto, and stirring was performed at room temperature for 12 hours. After confirming the reaction completion by TLC, water was added to extract only a MC layer, which was washed with sat. NaCl. A treatment with Na₂SO₄ was performed, and then filtration and concentration were performed. MC was added to the concentrated solution, and 6.4 mL of triethylamine (45.8 mmol) was added thereto. 2.4 mL of ethyl bromoacetate (22.0 mmol) was dissolved in MC and added dropwise slowly, and stirring was performed at room temperature for 12 hours. After confirming the reaction completion by TLC, water was added to extract only a MC layer. Water was removed by Na₂SO₄, the solution was concentrated, and purified by silica-column chromatography (eluent: EA:Hex = 1:1 v/v) to obtain Compound 5 which was a product in the form of transparent oil (3.79 g, 70%).

### [Preparation Example 2] Preparation of Compound 8 (Boc-Lys(Z)-OMe)

### Preparation of Compound 6

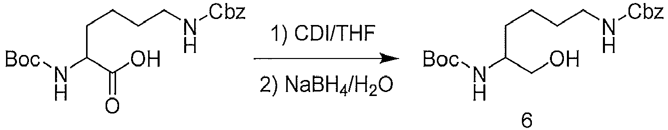

5.40 g of Boc-Lys(Z)-COOH (14.2 mmol) was added to a 250 mL 2-neck round bottom flask under nitrogen and dissolved in 100 mL of dry THF. 9.21 g of 1,1'-carbonyldiimidazole (56.8 mmol) was added all together, and stirring was performed at room temperature for 10 minutes. When bubbles did not occur any more, 2.68 g of NaBH₄ (71.0 mmol) was dissolved in 30 mL of distilled water at 0°C and was slowly added thereto, and stirring was performed for 30 minutes. After confirming the reaction completion by TLC, the solvent was concentrated. 200 mL of EA was added thereto, the solution was transferred to a separatory funnel, washed with 1 M HCl, and washed with saturated brine, and water was removed by sodium sulfate to concentrate the solution. Purification was performed with silica-column chromatography (eluent: EA:HEX = 1:1 v/v) to obtain Compound 6 which was a product in the form of transparent yellow oil (5.0 g, 96.2%).

### Preparation of Compound 7

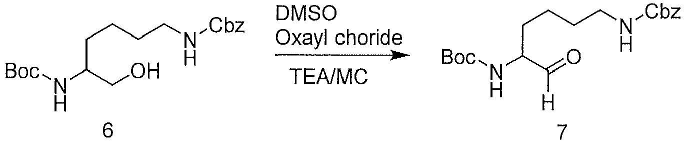

8.71 mL of oxayl chloride (17.4 mmol) was added to a 250 mL 2-neck round bottom flask using a syringe under nitrogen, and 20 mL of dry MC was added thereto. The temperature was lowered to -20°C for 5 minutes using NaCl, ice, and methanol, and a mixed solution of 2.47 mL of dry DMSO (34.8 mmol) and 5 mL of dry MC was added dropwise to a 100 mL round bottom flask. After 5 minutes, 5.80 g of Boc-Lys(Cbz)-OH (15.8 mmol) was dissolved in 20 mL of dry MC, and the solution was added to a reaction solution. After 15 minutes, 15.2 mL of DIEA (87.1 mmol) was added, stirring was performed for 5 minutes, the ice bath was removed, and TLC was confirmed (EA only, p-anisaldehyde). After reaction completion, the solution was washed with NaHCO₃, and the solution was concentrated to obtain Compound 7 which was a transparent oil (5.5 g, 95.3%).

### Preparation of Compound 8

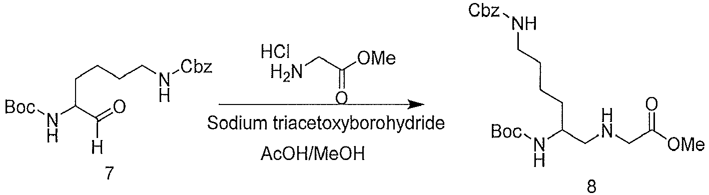

Compound 8 was prepared in the same manner as in a reference (FILBERT TOTSINGAN et. Al. CHIRALITY, 2009, 21, 245-253), and it was confirmed therefrom that Compound 8 was synthesized.

5.50 g of Boc-Lys(Cbz)-CHO (15.1 mmol) was added to a 250 mL of round bottom flask, 50.0 mL of methanol was added thereto, and 2.84 g of Gly-OMe (22.6 mmol) was added thereto. 1.30 mL of acetic acid (22.6 mmol) and 3.94 mL of N,N-diisopropylethylamine (22.6 mmol) were added thereto at 0°C, 9.60 g of sodium triacetoxyborohydride (45.3 mmol) was added, and stirring was performed at 0°C for 2 hours, and then at room temperature overnight. By TLC (eluent: EA only, p-anisaldehyde) confirmation, it was confirmed whether the starting material reacted, the solution was concentrated at the end, EA was added, the solution was washed with NaHCO₃, concentrated, and purified with silica-column chromatography (eluent: EA:HEX = 1:1 v/v), thereby obtaining a product in the form of a transparent oil (4.5 g, 68.2%).

### [Preparation Example 3] Preparation of Compound 11 (Boc-Glu(OcHex)-OMe)

### Preparation of Compound 9

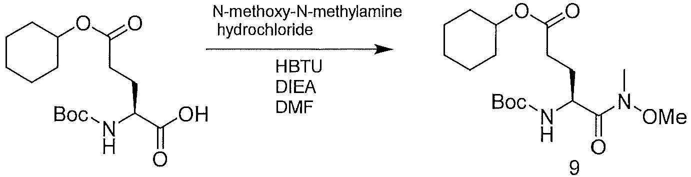

20.0 g of Boc-Glu(OcHex)-COOH (60.7 mmol) was added to a 500 mL of 1-neck round bottom flask, and was dissolved in 150 mL of dry N,N-dimethylformamide (DMF). 22.3 g of O-(1H-benzotriazol-1-yl-N,N,N',N'-tetramethyluroniumhexafluorophosphate) (HBTU) (58.3 mmol) was added thereto. 23.3 mL of N,N-diisopropylethylamine (DIEA) (133.6 mmol) was added at 0°C, 7.11 g of N-methoxy-N-methylamine hydrochloride (72.9 mmol) was added thereto, and stirring was performed at room temperature overnight. By TLC (eluent: 3% AcOH, 5% MeOH/MC, ninhydrin) confirmation, it was confirmed whether the starting material reacted, the solution was concentrated at the end, 150 ml of ethyl acetate (EA) was added, and the solution was washed with 0.5 N HCl, saturated NaHCO₃, and 1 N KHSO₄. Sodium sulfate was added to the organic layer to remove water from the organic layer, which was concentrated to obtain Compound 9 which was a product in the form of a transparent colorless oil (21 g, 92.9%) .

### Preparation of Compound 10

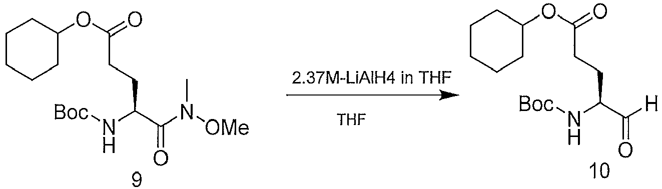

21.0 g of Compound 9 (56.4 mmol) was added to a 1 L 3-neck round bottom flask under nitrogen, and was dissolved in 210 mL of dry tetrahydrofuran (THF). 26.2 g of 2.37 M lithium aluminum hydride (62.0 mmol) was added thereto -78°C for 20 minutes and then stirring was performed for 1 hour. By TLC (eluent: EA:Hex = 1:1, ninhydrin) confirmation, it was confirmed whether the starting material reacted, 600 mL of ethyl acetate was added at the end, 210 mL of 1 M KHSO₄ was added thereto for 10 minutes, and stirring was performed for 10 minutes. The organic layer was washed with 1M KHSO₄ and saturated NaHCO₃, water was removed by sodium sulfate, and the solution was concentrated, thereby obtaining Compound 10 which was a product in the form of a transparent yellow oil (18.5 g, 88.1%).

### Preparation of Compound 11

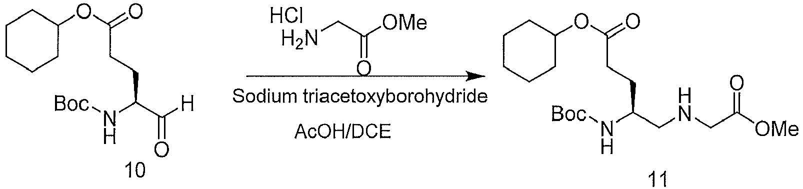

18.5 g (59.0 mmol) of Compound 10 was added to a 500 mL round bottom flask, 130 mL of dichloroethane was added thereto, and glycine methyl ester hydrochloride (HCl. Gly-OMe) was added thereto. 3.38 mL of acetic acid (59.0 mmol) and 15.4 mL of N,N-diisopropylethylamine (88.5 mmol) were added thereto at 0°C, 18.8 g of sodium triacetoxy borohydride (88.5 mmol) was added thereto, and stirring was performed for 30 minutes. By TLC (eluent: EA:Hex = 1:4, developed twice, ninhydrin) confirmation, it was confirmed whether the starting material reacted, 130 mL of saturated NaHCO₃ was added at 0°C at the end, and stirring was performed for 10 minutes. The organic layer was washed with saturated NaHCO₃ and saturated brine, water was removed with sodium sulfate, solution was concentrated, and purification was performed with silica-column chromatography (eluent: EA:Hex = 1:1 v/v), thereby obtaining Compound 11(Boc-Glu(OcHex)-OMe) which was a product in the form of a transparent oil (14 g, 61.4%).

### [Preparation Example 4] Preparation of Compound 12 (Boc-Lys(Z)-NH₂)

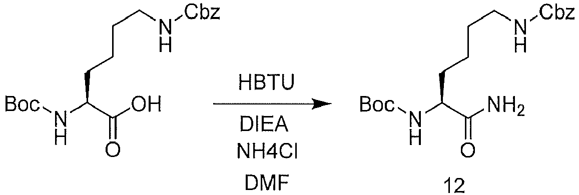

### Preparation of Compound 12

20.0 g of Boc-Lys(Cbz)-COOH (52.6 mmol) was added to a 1 L round bottom flask, and was dissolved in 200 mL of N,N-dimethylformamide (DMF). 29.9 g of O-(1H-Benzotriazol-1-yl-N,N,N',N'-tetramethyluroniumhexafluorophosphate) (HBTU) (78.9 mmol) was added thereto, 5.62 g of NH₄Cl (105.1 mmol) was added thereto, and 36.6 mL of N,N-diisopropylethylamine (DIEA) (210.3 mmol) was added dropwise at 0°C. After stirring for about 30 minutes, TLC was used to confirm reaction completion, dichloromethane (200 mL) was added thereto, and the solution was washed with 5% NaHCO₃ once and with 0.5 M HCl once using a separatory funnel. As the organic layer gradually became cloudy, a solid was produced, which was filtered to obtain Compound 12 (Boc-Lys(Z)-NH₂) (19.9 g, 99.9%).

### [Example 1] Production of PNA monomer

### Preparation of Compound 13-3 (Boc-aeg-A(Z)-OEt)

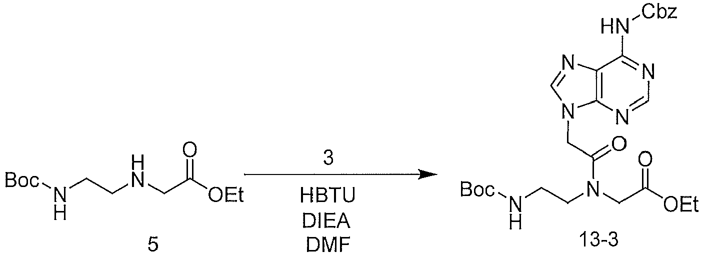

20.0 g of Compound 3 (61.1 mmol) was added to a 3 L round bottom flask, and was dissolved in 1 L of N,N-dimethylformamide (DMF). 34.8 g of O-(1H-benzotriazol-1-yl-N,N,N',N'-tetramethyluroniumhexafluorophosphate) (HBTU) (122 mmol) was added thereto, 15.1 g of Compound 5 (61.1 mmol) was added thereto, 31.9 mL of N,N-diisopropylethylamine (DIEA) (183 mmol) was added dropwise at 0°C, and then stirring was performed for 30 minutes. TLC (developing solution: 5% MeOH/MC) was used to confirm reaction completion, and 1 L of dichloromethane (MC) was added thereto. The organic layer was washed with saturated NaHCO₃ and 0.5 M HCl, sodium sulfate was added to remove water, and the solution was concentrated. Purification was performed with silica-column chromatography (eluent: 5% MeOH/MC) to obtain Compound 13 (Boc-A(Z)-OEt) which was a white solid product (30.0 g, 88.4%).

### Preparation of Compound 14-3 (Boc-aeg-A(Z)-OH)

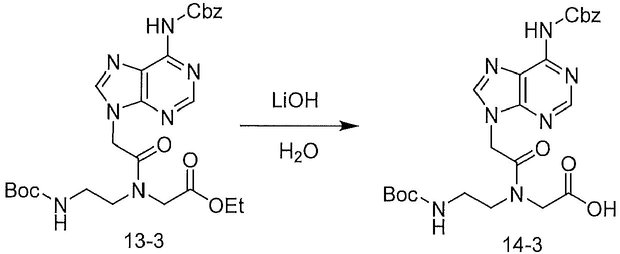

15.0 g of Compound 13-3 (27.0 mmol) was added to a 500 mL round bottom flask and was dissolved in 270 mL of 0.5 N LiOH (135 mmol), stirring was performed for about 30 minutes, and TLC (developing solution: 5% MeOH/MC) was used to confirm reaction completion. 0.5 M HCl was added dropwise at 0°C to lower the pH to 2-3, and a produced solid was filtered through a glass filter, washed with water several time, and dried with P₂O₅, thereby obtaining Compound 14-3 (Boc-A(Z)-OH) which was a product in the form of a white solid (14.1 g, 99.0%).

### Preparation of Compound 15-1 (NH₂-aeg-A(Z)-OEt)

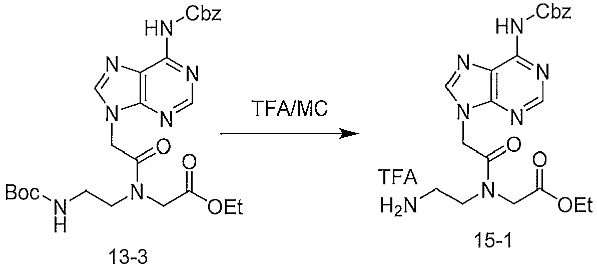

15.0 g of Compound 13-3 (27.0 mmol) was added to a 250 mL round bottom flask and was dissolved in 135 mL of 50% TFA/MC at 0°C, and stirring was performed for 30 minutes. TLC (developing solution: 5% MeOH/MC) was used to confirm reaction completion. The solution was added dropwise to an excessive amount of diethyl ether to cause precipitation, which was filtered through a glass filter, washed with diethyl ether several times, and dried, thereby obtaining Compound 15-1 (NH₂-A(Z)-OEt) which was a product in the form of a white solid (14.5 g, 94.3%).

### [Example 2] Production of PNA monomer

PNA monomer 14-1 (Boc-aeg-T-OH) and PNA monomer 15-1 (NH₂-aeg-T-OEt) were produced in the same manner as in Example 1, except that Compound 1 was used instead of Compound 3 in the preparation of Compound 13-3 of Example 1.

### [Example 3] Production of PNA monomer

PNA monomer 14-2 (Boc-aeg-C(Z)-OH), PNA monomer 14-4 (Boc-aeg-G(Z)-OH), and PNA monomer 15-4 (NH₂-aeg-G(Z)-OEt) were produced in the same manner as in Example 1, except that each of Compound 2 and 4 was used instead of Compound 3 in the preparation of Compound 13-3 of Example 1.

### [Example 4] Production of PNA monomer

PNA monomer 14-5 (Boc-Lys(Z)-C(Z)-OH) and PNA monomer 15-6 (NH₂-Lys (Z) -NH₂) were produced in the same manner as in Example 1, except that Compound 8 was used instead of Compound 5 and Compound 2 was used instead of Compound 3 in the preparation of Compound 13-3 of Example 1.

### [Example 5] Production of PNA monomer

PNA monomer 15-3 (NH₂-Glu(OcHex)-T-OMe) was produced in the same manner as in Example 1, except that Compound 11 was used instead of Compound 5 and Compound 1 was used instead of Compound 3 in the preparation of Compound 13-3 of Example 1.

### [Example 6] Production of PNA monomer

PNA monomer 15-4 (NH₂-Glu(OcHex)-C(Z)-OMe) and PNA monomer 15-5 (NH₂-Glu(OcHex)-G(Z)-OMe) were prepared, respectively, in the same manner as in Example 5, except that each of Compound 2 and 4 was used instead of Compound 1.

### [Example 7] Preparation of PNA dimer 17-1 (Boc-AA-OH)

### Preparation of Compound 16-1 (Boc-AA-OEt)

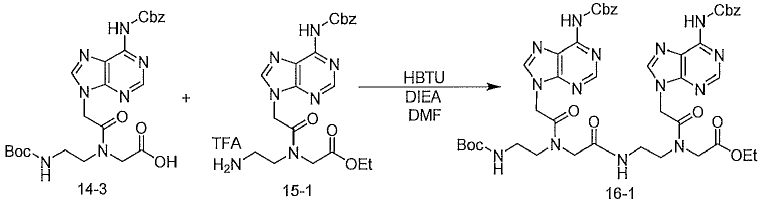

13.0 g of Compound 14-3 (24.6 mmol) was added to a 2 L round bottom flask and was dissolved in 650 mL of dry N,N-dimethylformamide (DMF), and 14.0 g of O-(1H-Benzotriazol-1-yl-N,N,N',N'-tetramethyluroniumhexafluorophosphate) (HBTU) (37.0 mmol) was added thereto. 14.0 g of Compound 15-1 (24.6 mmol) was added thereto, 21.5 mL of N,N-diisopropylethylamine (DIEA) (123 mmol) was added dropwise at 0°C, and stirring was performed for 30 minutes. TLC (developing solution: 15% MeOH/MC) was used to confirm reaction completion, and 650 mL of dichloromethane (MC) was added thereto. The organic layer was washed with saturated NaHCO₃ and 0.5 M HCl, sodium sulfate was added to remove water, and the solution was concentrated. Purification was performed with silica-column chromatography (eluent: 10% MeOH/MC) to obtain a white solid product, Boc-AA-OEt (21.0g, 87.9%).

### Preparation of Compound 17-1 (Boc-AA-OH)

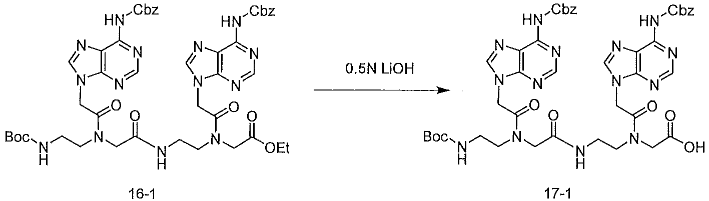

21.0 g of Compound 16-1 (21.7 mmol) was added to a 500 mL round bottom flask and was dissolved in 217 mL of 0.5 N LiOH (108 mmol) at 0°C, and stirring was performed for 30 minutes. TLC (developing solution: 10% MeOH/MC) monitoring was performed to complete the reaction. 1 M HCl was added dropwise at 0°C to lower the pH to 2-3 to produce a solid, which was filtered through a glass filter, washed with water several times, and then recrystallized with MeOH/ether, thereby obtaining Compound 17-1 (Boc-AA-OH) which was a white product (19.7 g, 96.6%).

### [Example 8] Production of PNA dimer

PNA dimer 16-2 (Boc-C"A-OEt), PNA dimer 17-2(Boc-CC^-OH) , PNA dimer 17-3 (Boc-TT^-OH), and PNA dimer 17-4 (Boc-GG-OH) were produced, respectively, in the same manner as in Example 7, the PNA monomer was changed, respectively, instead of Compounds 14-3 and 15-3. At this time, base" was NH₂-γLys(Z)-base-OMe, and base^ was NH₂-γGlu(OcHex)-base-OMe.

### [Example 9] Production of PNA dimer 18-1 (NH2-C"A-OEt)

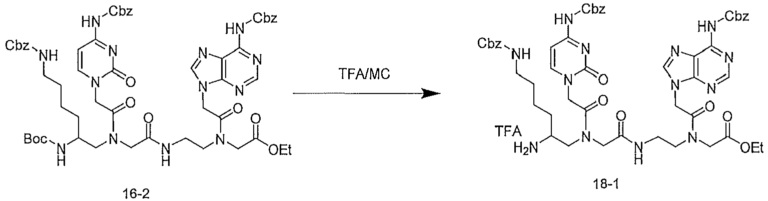

20.0 g of Compound 16-2 (Boc-C"A-OEt) (17.4 mmol) was added to a 250 mL round bottom flask and was dissolved in 180 mL of 50% TFA/MC at 0°C, and stirring was performed for 30 minutes. TLC (developing solution: 7% MeOH/MC) was used to complete the reaction. The solution was added dropwise to an excessive amount of diethyl ether to cause precipitation, which was filtered through a glass filter and washed with diethyl ether, thereby obtaining NH₂-C"A-OEt which was a white solid product (19.2 g, 94.9%).

### [Example 10] Preparation of OK linker (Compound 18-2, NH₂-OK-NH₂)

### Preparation of Compound 16-6 (Boc-OK-NH₂)

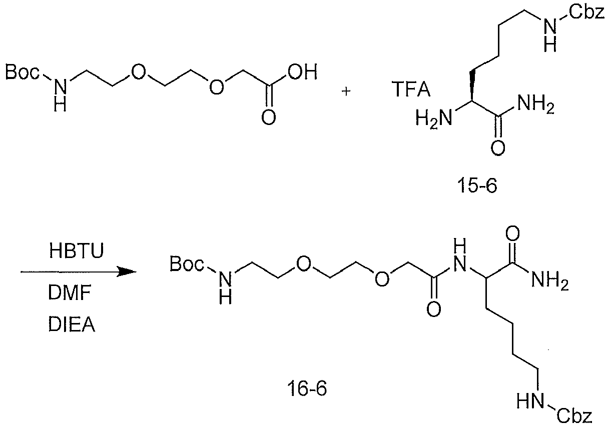

13.0 g of 3,6,11-trioxa-9-azatridecanoic acid, 12,12-dimethyl-10-oxo- 97% (Boc-O-OH) (49.4 mmol) was added to a 2 L round bottom flask and was dissolved in 650 mL of dry N,N-dimethylformamide (DMF), and 22.5 g of O-(1H-Benzotriazol-1-yl-N,N,N',N'-tetramethyluroniumhexafluorophosphate) (HBTU) (59.3 mmol) was added thereto. 19.4 g of Compound 15-6 (49.4 mmol) was added thereto, 21.5 mL of N,N-diisopropylethylamine (DIEA) (123 mmol) was added dropwise at 0 °C, and stirring was performed for 30 minutes. TLC (developing solution: 7% MeOH/EA) was used to confirm reaction completion, and 650 mL of dichloromethane (MC) was added thereto. The organic layer was washed with saturated NaHCO₃ and 0.5 M HCl, sodium sulfate was added to remove water, and the solution was concentrated. Purification was performed with silica-column chromatography (eluent: 7% MeOH/EA) to obtain Boc-OK-NH₂ in the form of a transparent colorless oil (21.0g, 81.1%).

### Preparation of Compound 18-2 (NH₂-OK-NH₂)

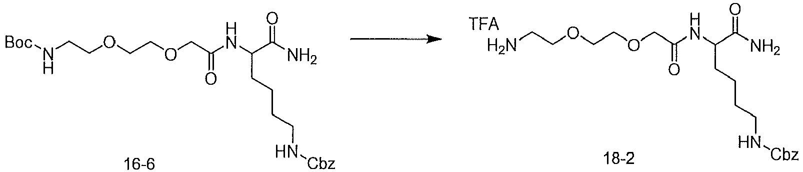

21.0 g of Compound 16-6 (Boc-OK-NH₂) (40.0 mmol) was added to a 250 mL round bottom flask and was dissolved in 180 mL of 50% TFA/MC at 0°C, and stirring was performed for 30 minutes. TLC (developing solution: 7% MeOH/EA) was used to complete the reaction. The solution was added dropwise to an excessive amount of diethyl ether to cause precipitation, which was centrifuged to obtain a product in the form of oil, the product was dissolved in dichloromethane and the solvent was concentrated, thereby obtaining NH₂-OK-NH₂ which was a product in the form of a colorless transparent oil (19.2 g, 89.1%).

### [Example 11] Preparation of PNA trimer 19-1 (Boc-AAG^-OMe)

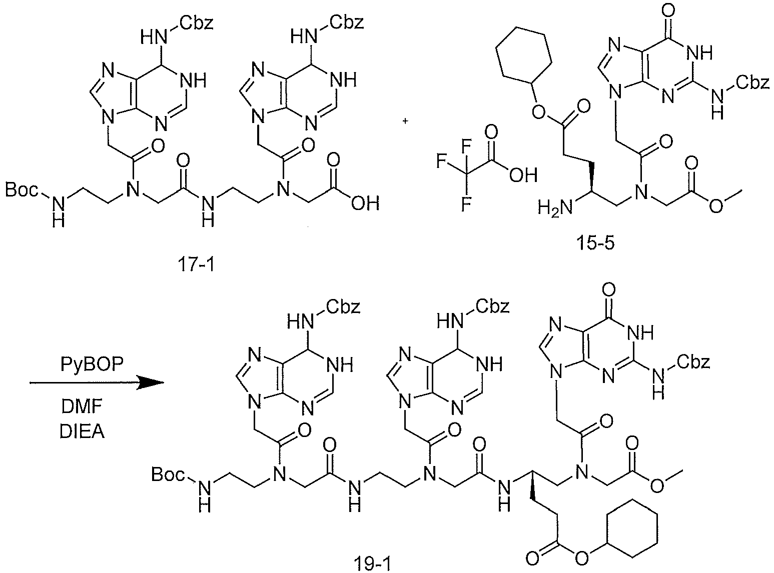

15.2 g of Compound 17-1 (16.2 mmol) was added to a 2 L round bottom flask, and was dissolved in 750 mL of dry N,N-dimethylforamide (DMF). 12.6 g of benzotriazole-1-yl-oxytripyrrolidinophosphate (PyBOP) (24.2 mmol) was added thereto, thereby obtaining 11.7 g of Compound 15-5 (16.2 mmol). 56.3 mL of N,N-diisopropylethylamine(DIEA) (323 mmol) was added thereto at 0°C, stirring was performed for about 30 minutes, and TLC (developing solution: 20% MeOH/MC) was used to confirm reaction completion. 300 mL of dichloromethane was added thereto, the organic layer was washed with a saturated NaHCO₃, 0.5 M HCl solution, water was removed with sodium sulfate, and the solution was concentrated, thereby obtaining a product in the form of a transparent yellow oil. The product was recrystallized with MeOH/ether to obtain Compound 19-1 (Boc-AAG^-OMe) which was a white solid compound (21.3 g, 85.9%).

### [Example 12] Preparation of PNA trimer 19-2 (Boc-CC"A-OEt)

PNA trimer 19-2 (Boc-CC"A-OEt) was prepared in the same manner as in Example 11, except that PNA monomer 14-2 and PNA dimer 18-1 were used instead of PNA dimer 17-1 and PNA monomer 15-5. At this time, Base" was NH₂-γLys(Z)-base-OMe.

### [Example 13] Preparation of PNA trimer 20-1 (Boc-AAG^-OH)

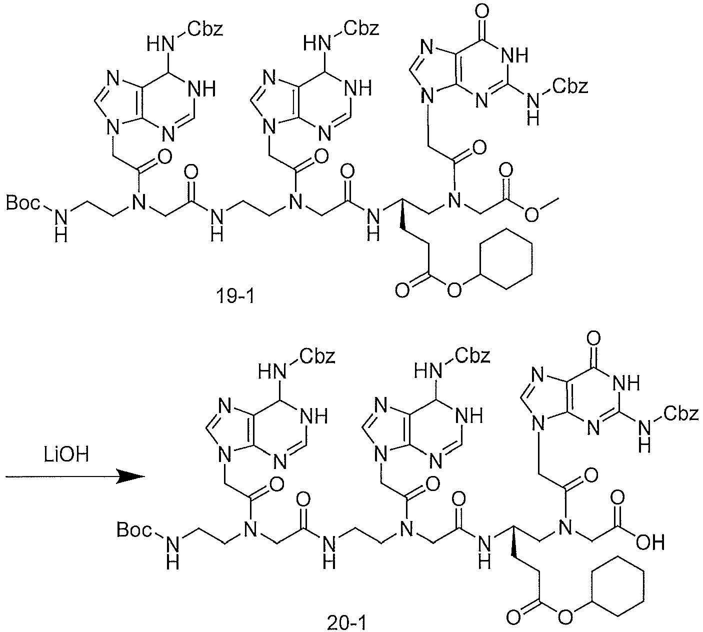

20.3 g of Compound 19-1 (13.2 mmol) was added to a 1 L round bottom flask and was dissolved in 100 mL of 1,4-dioxane. 132 mL of 0.5 M LiOH (66.1 mmol) was added dropwise at 0°C and stirring was performed for about 30 minutes. TLC (developing solution: 20% MeOH/MC) was used to confirm reaction completion. 0.5 M HCl was added dropwise at 0°C to lower the pH to 2-3 to produce a solid, which was filtered through a glass filter, washed with water, and then recrystallized with MeOH/ether, thereby obtaining Compound 20-1 (Boc-AAG^-OH) which was a white solid product (18.3 g, 90.8%) .

### [Example 14] Preparation of PNA trimer 21-1(NH₂-CC"A-OEt)

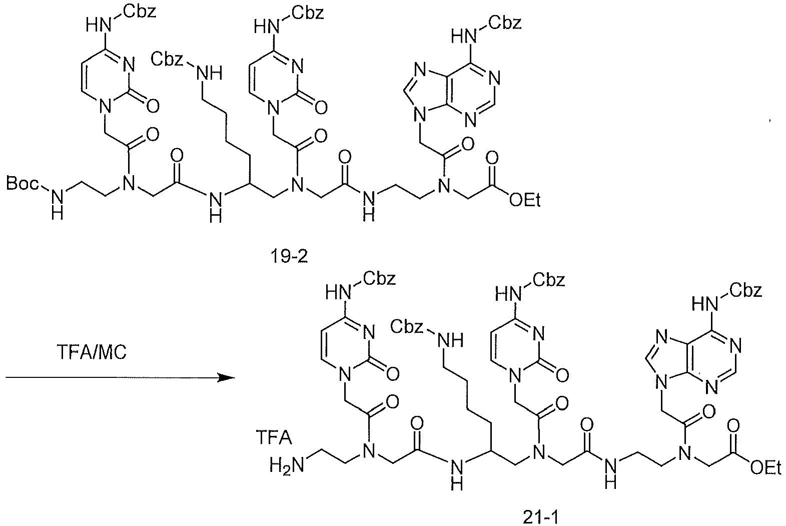

25.3 g of Compound 19-2 (16.5 mmol) was added to a 250 mL round bottom flask and was dissolved in 230 mL of 50% TFA/MC at 0°C, stirring was performed for 30 minutes, and TLC (developing solution: 20% MeOH/MC) was used to complete the reaction. The solution was added dropwise to an excessive amount of diethyl ether to cause precipitation, which was filtered through a glass filter and washed with diethyl ether, thereby obtaining Compound 21-1 (NH₂-CC"A-OEt) which was a white solid product (23.8 g, 93.2%).

### [Example 15] Preparation of PNA 23-1 (NH₂-GGOK-NH₂)

### Preparation of Compound 22-1 (Boc-GGOK-NH₂)

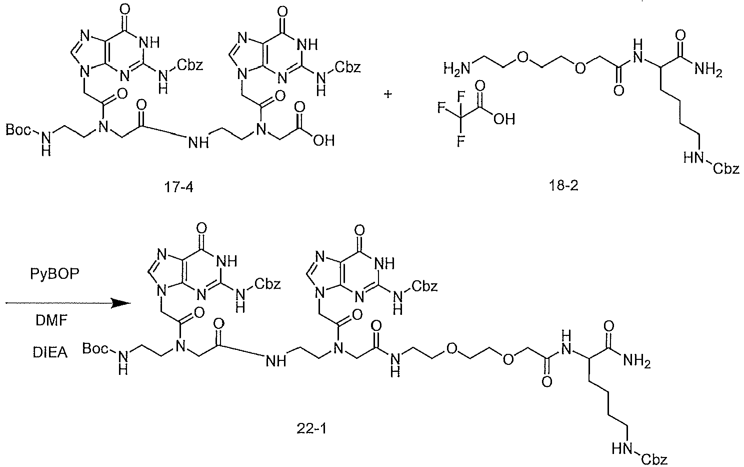

13.8 g of Compound 17-4 (14.2 mmol) was added to a 1 L round bottom flask, and was dissolved in 650 mL of dry N,N-dimethylforamide (DMF). 11.1 g of benzotriazole-1-yl-oxytripyrrolidinophosphate (PyBOP) (21.4 mmol) was added thereto, thereby obtaining 7.67 g of Compound 18-2 (14.2 mmol). 49.6 mL of N,N-diisopropylethylamine (DIEA) (285 mmol) was added thereto at 0°C, stirring was performed for about 30 minutes, and TLC (developing solution: 20% MeOH/MC) was used to confirm reaction completion. 650 mL of dichloromethane (MC) was added, the organic layer was washed with a saturated NaHCO₃, 0.5 M HCl solution, water was removed by sodium sulfate, and the concentrated product in the form of a transparent colorless oil was recrystallized with MeOH/ether, thereby obtaining Compound 22-1 (Boc-GGOK-NH₂) which was a white solid compound (17.7 g, 90.4%).

### Preparation of Compound 23-1 (NH₂-GGOK-NH₂)

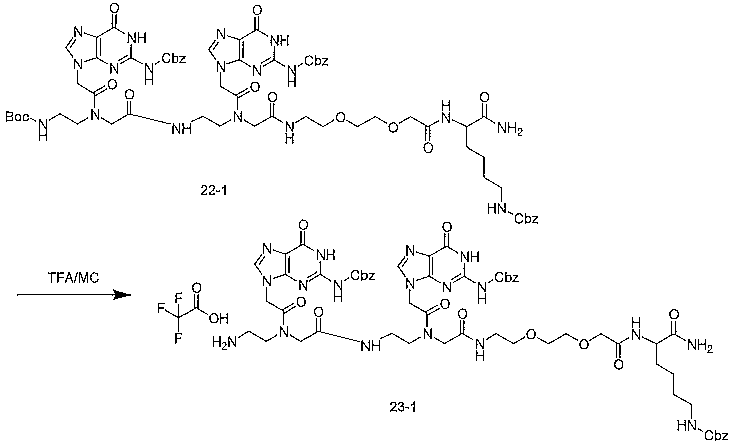

16.7 g of Compound 22-1 (12.1 mmol) was added to a 250 mL round bottom flask and was dissolved in 140 mL of 50% TFA/MC at 0°C, stirring was performed for 30 minutes, and TLC (developing solution: 20% MeOH/MC) was used to complete the reaction. The solution was added dropwise to an excessive amount of diethyl ether to cause precipitation, which was filtered through a glass filter and washed with diethyl ether, thereby obtaining Compound 23-1 (NH₂-GGOK-NH₂) which was a white solid product (16.0 g, 94.8%).

### [Example 16] Preparation of PNA pentamer 25-1 (Boc-CC^CC"A-OH)

### Preparation of Compound 24-1 (Boc-CC^CC"A-OEt)

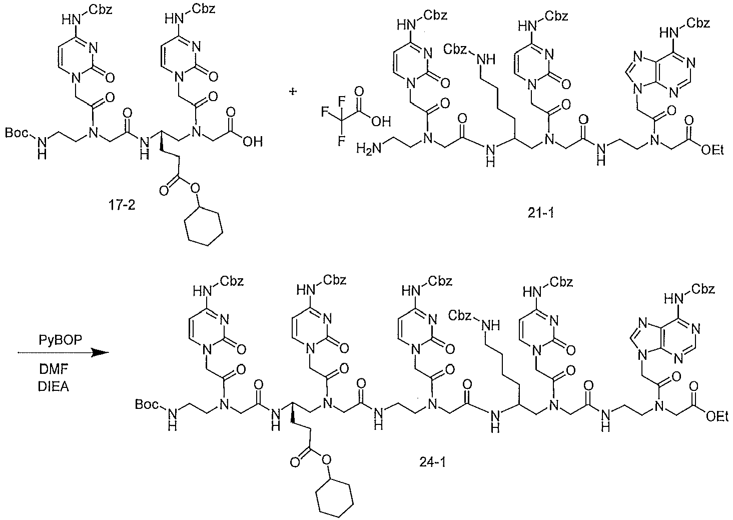

6.13 g of Compound 17-2 (5.88 mmol) was added to a 1 L round bottom flask, and was dissolved in 300 mL of dry N,N-dimethylforamide (DMF). 4.59 g of benzotriazole-1-yl-oxytripyrrolidinophosphate (PyBOP) (8.82 mmol) was added thereto, 9.08 g of Compound 21-1 (5.88 mmol) was added thereto, and 20.5 mL of N,N-diisopropylethylamine (DIEA) (118 mmol) was added dropwise at 0°C. Stirring was performed for about 30 minutes, TLC (developing solution: 5% AcOH, 10% MeOH/MC) was used to confirm reaction completion, 300 mL of dichloromethane (MC) was added thereto, and the organic layer was washed with saturated NaHCO₃, 0.5 M HCl. Water was removed from the organic layer by sodium sulfate, and the solution was concentrated to obtain a product, which was recrystallized with MeOH/ether to obtain Compound 24-1 (Boc-CC^CC"A-OEt) which was a white solid product (13.2 g, 91.4%).

### Preparation of Compound 25-1 (Boc-CCACC"A-OH)

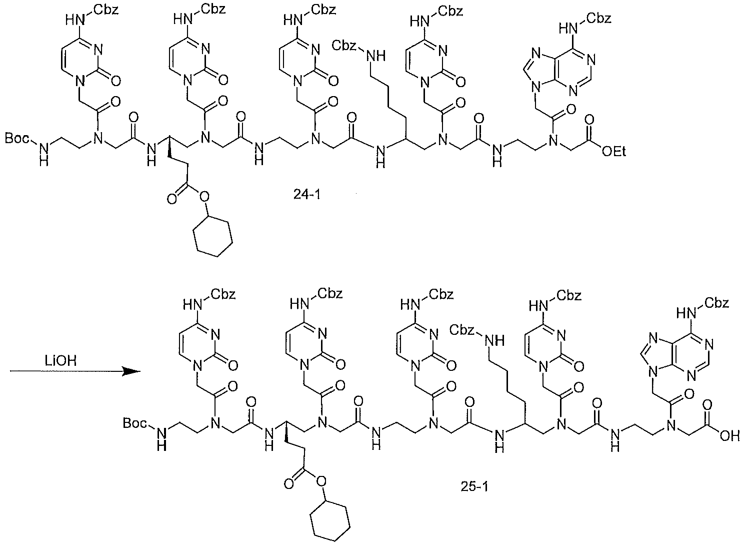

13.0 g of Compound 24-1 (5.29 mmol) was added to a 500 mL round bottom flask and was dissolved in 71.5 mL of 1,4-dioxane. 52.9 mL of 0.5 N LiOH (26.5 mmol) was added at 0°C and stirring was performed for about 30 minutes. TLC (developing solution: 5% AcOH, 20% MeOH/MC) was used to confirm reaction completion, and the pH was lowered with 0.5 M HCl to 2-3 to produce a solid, which was filtered through a glass filter, washed with water, and recrystallized with MeOH/ether. The produced solid was filtered through a glass filter, washed with diethyl ether, and dried, thereby obtaining Compound 25-1 (Boc-CC^CC"A-OH) which was a product in the form of a white solid (12.1 g, 94.2%).

### [Example 17] Preparation of PNA hexamer 27-1 (Boc-AAGACC"A-OH)

### Preparation of Compound 26-1 (Boc-AAGACC"A-OEt)

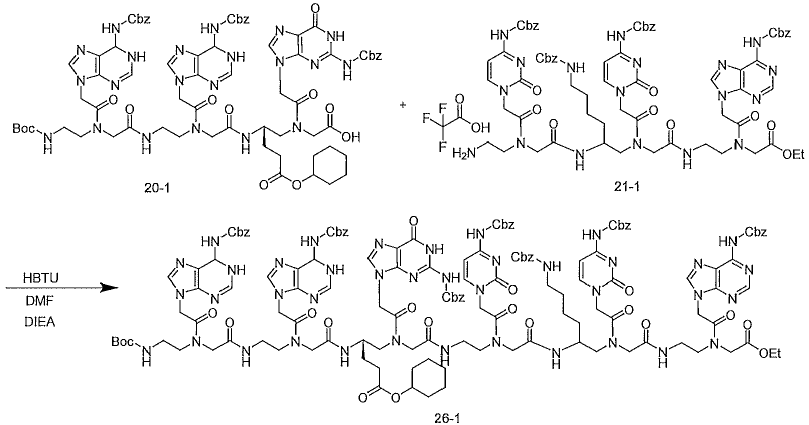

11.7 g of Compound 20-1 (7.66 mmol) was added to a 2 L round bottom flask, and was dissolved in 550 mL of dry N,N-dimethylforamide (DMF). 4.36 g of O-(1H-Benzotriazol-1-yl-N,N,N',N'-tetramethyluroniumhexa fluorophosphate) (HBTU) (11.5 mmol) was added thereto, and then 11.8 g of Compound 21-1 (7.66 mmol) was added thereto. 26.7 mL of N,N-diisopropylethylamine (DIEA) (153 mmol) was added at 0°C, and stirring was performed for 30 minutes. TLC (developing solution: 5% AcOH, 15% MeOH/MC) was used to confirm reaction completion, 550 mL of dichloromethane was added thereto, the organic layer was washed with a saturated NaHCO₃, 0.5 M HCl solution, water was removed with sodium sulfate, and the solution was concentrated, thereby obtaining a product in the form of a transparent yellow oil. The compound was recrystallized with MeOH/ether to obtain Compound 26-1 (Boc-AAG^CC"A-OEt) which was a white solid compound (yield: 18.2 g, 81.0%).

### Preparation of Compound 27-1 (Boc-AAG^CC"A-OH)

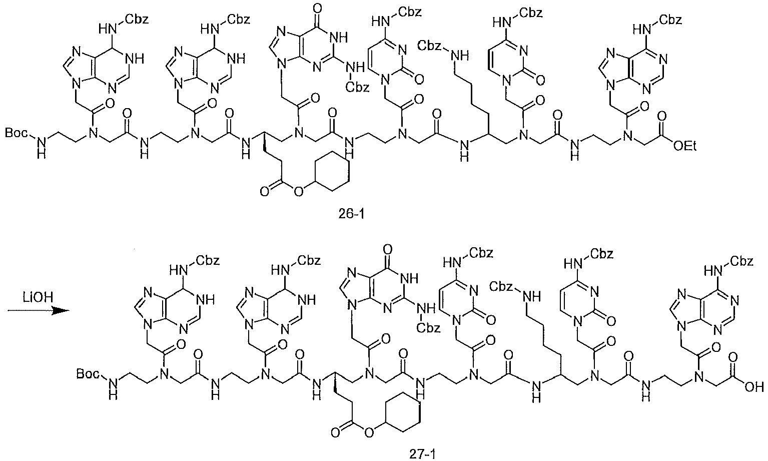

18.2 g of Compound 26-1 (6.20 mmol) was added to a 500 mL round bottom flask and was dissolved in 182 mL of 1,4-dioxane. 62.0 mL of 0.5 N LiOH was added dropwise at 0°C and stirring was performed for 30 minutes. TLC (developing solution: 5% ArOH, 15% MeOH/MC) monitoring was performed to complete the reaction. pH was lowered to pH 2-3 with 1 M HCl at 0°C to produce a solid, which was filtered, washed with water, dried, and recrystallized with MeOH/ether, thereby obtaining Compound 27-1 which was a white compound (15.1 g, 83.8%) [Prep purification was performed].

[Example 18] Preparation of PNA tetramer linker compound 28-1 (TFA.NH₂-TT^GGOK-NH₂)

### Preparation of Compound 26-2 (Boc-TT^GGOK-NH₂)

Compound 26-2 (Boc-TT^GGOK-NH₂) was prepared in the same manner as in Example 17, except that PNA trimers 17-3 and 23-1 were used instead of PNA trimer 20-1 and PNA trimer 21-1. At this time Base^ was NH₂-γGlu(OcHex)-base-OMe.

### Preparation of Compound 28-1 (TFA.NH₂-TT^GGOK-NH₂)

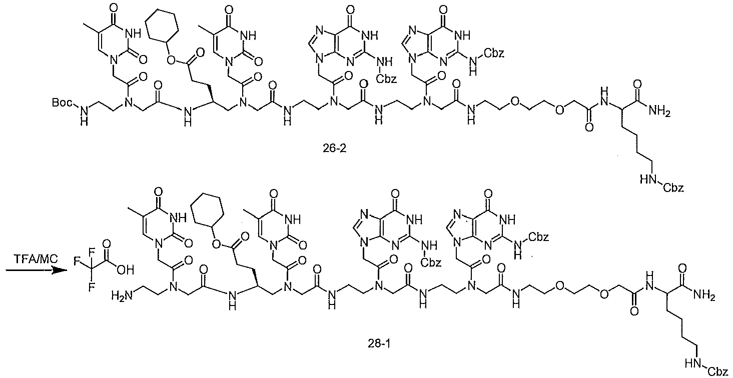

15.5 g of Compound 26-2 (7.52 mmol) was added to a 250 mL round bottom flask and was dissolved in 140 mL of 50% TFA/MC at 0°C, and then stirring was performed for 30 minutes. TLC (developing solution: 20% MeOH/MC) monitoring was performed to complete the reaction. After reaction completion, the solution was added dropwise to an excessive amount of diethyl ether at 0°C to cause precipitation. After filtration, Compound 28-1 (TFA.NH₂-TT^GGOK-NH₂) which was a product in the form of a white solid (14.6 g, 94.3%) was obtained.

### [Example 19] Preparation of PNA decamer linker compound 30-1 (NH₂-CC^CC"AATT^GGOK-NH₂)

### Preparation of Compound 29-1 (Boc-CC^CC"ATT^GGOK-NH₂)

5.76 g of Compound 25-1 (2.37 mmol) was added to a 1 L round bottom flask and was dissolved in 250 mL of dry N,N-dimethylformamide (DMF), and 1.85 g of benzotriazole-1-yl-oxytripyrrolidinophosphate (PyBOP) (3.56 mmol) was added thereto. 4.92 g of Compound 28-1 (2.37 mmol) was added thereto, 12.0 mL of diisopropylethylamine (DIEA) (68.8 mmol) was added dropwise at 0°C, and stirring was performed for 30 minutes. The thus-produced crude PNA oligomer was subjected to reaction completion by HPLC (Agilent 1100, 10% MeCN 70% for 20 min 100% for 25min) and a crude purity was measured. 250 mL of MC was added thereto, the organic layer was washed with a saturated NaHCO₃, 0.5 M HCl solution, water was removed by sodium sulfate, and the solution was concentrated to obtain a product in the form of a transparent yellow oil, which was recrystallized with MeOH/ether to produce Compound 29-1 (Boc-CC^CC"ATT^GGOK-NH₂) in the form of a white solid (8.32 g, 80.3%)

### Preparation of Compound 30-1 (TFA.NH2-CC^CC"ATT^GGOK-NH2)

8.32 g of Compound 30-1 (1.90 mmol) was added to a 250 mL round bottom flask and was dissolved in 140 mL of 50% TFA/MC at 0°C, and then stirring was performed for 30 minutes. The thus-produced crude PNA oligomer was subjected to reaction completion by HPLC (Agilent 1100, 10% MeCN 70% for 20 min 100% for 25min) and a crude purity was measured. After reaction completion, the solution was added dropwise to an excessive amount of diethyl ether at 0°C to cause precipitation. After filtration, Compound 30-1 (TFA.NH₂-CC^CC"ATT^GGOK-NH₂) which was a product in the form of a white solid (7.31 g, 87.6%) was obtained. [Prep purification was performed.]

### [Example 20] Preparation of PNA pentadecamer 31-1 (Boc-AAG^CC"ACC^CC"ATT^GGOK-NH₂)

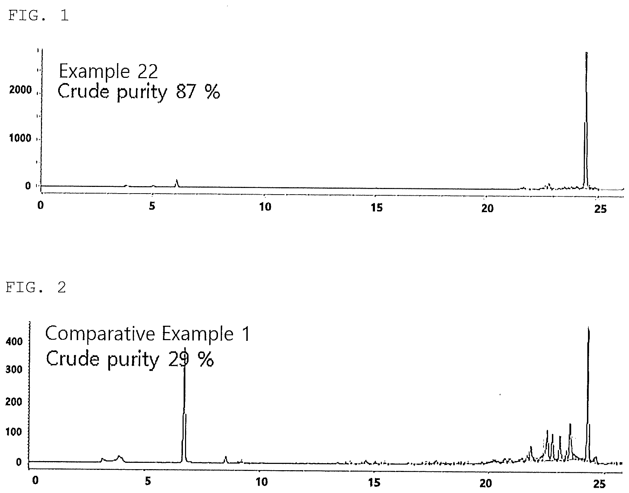

3.54 g of Compound 20-1 (1.22 mmol) was added to a 500 mL round bottom flask and was dissolved in 350 mL of dry dimethylformamide (DMF), and 0.693 g of O-(1H-Benzotriazol-1-yl-N,N,N' ,N'-tetramethyluronium hexafluorophosphate) (HBTU) (1.83 mmol) was added thereto. 5.34 g of Compound 30-1 (1.22 mmol) was added thereto, 4.24 mL of diisopropylethylamine (DIEA) (24.4 mmol) was added at 0°C, and stirring was performed for 30 minutes. The crude purity of the thus-produced crude PNA oligomer was measured by HPLC (Agilent 1100, 10% MeCN 70% for 20 min 100% for 25min), and the results are shown in FIG. 1.

As seen in FIG. 1, the measured crude purity was 87%.

### [Comparative Example 1] Preparation of Compound 31-1 (Boc-AAG^CC"ACC^CC"ATT^GGOK-NH₂)

1.00 g of Compound 20-1 (0.344 mmol) was added to a 500 mL round bottom flask and was dissolved in 50 mL of dry dimethylformamide (DMF), 0.196 g of O-(1H-benzotriazol-1-yl-N,N,N',N'-tetramethyluronium hexafluorophosphate) (HBTU) (0.516 mmol) was added thereto, and 0.6 mL of diisopropylethylamine (DIEA) (3.44 mmol) was stirred at room temperature for 10 minutes. 1.51 g of Compound 30-1 (0.344 mmol) was dissolved in 50 mL of dry dimethylformamide (DMF), 0.6 mL of diisopropylethylamine (DIEA) (3.44 mmol) was added thereto, and stirring was performed at 0°C for 10 minutes. The thus-produced solution was mixed at 0°C, stirring was performed for 30 minutes, the crude purity was measured by HPLC (Agilent 1100, 10% MeCN 70% for 20 min 100% for 25min), and the results are shown in FIG. 2.

As seen in FIG. 2, it is recognized that the crude purity was significantly different depending on the order of addition of the amine compound.

### [Example 21] Production of PNA oligomer

PNA oligomer 31-1 (Boc-AAG^CC"ACC^CC"ATT^GGOK-NH₂) was produced in the same manner as in Example 20, except that 30 mL of dry dimethylformamide (DMF) was used.

## Claims

1. A method for producing a PNA oligomer, the method comprising: reacting PNA blocks with each other in a solution phase to produce a PNA oligomer,
wherein the PNA block is two or more identical or different PNA monomers bound to each other.

2. The method for producing a PNA oligomer of claim 1, wherein the method includes:
in the presence of a coupling reagent or a solvent,
mixing a first PNA dimer represented by the following Chemical Formula 1, a first PNA trimer represented by the following Chemical Formula 2, or a first PNA tetramer represented by the following Chemical Formula 3 with a second PNA dimer represented by the following Chemical Formula 4, a second PNA trimer represented by the following Chemical Formula 5, or a second PNA tetramer represented by the following Chemical Formula 6, and then adding an amine compound to produce a PNA oligomer represented by the following Chemical Formulae 7 to 9: 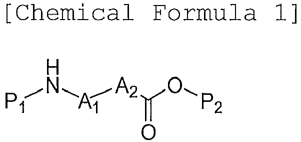 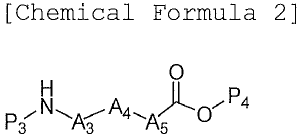 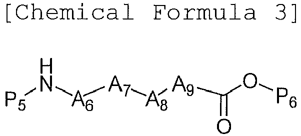 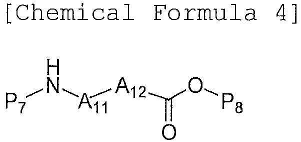 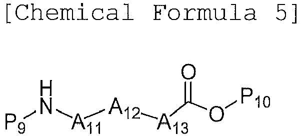 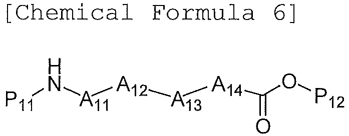 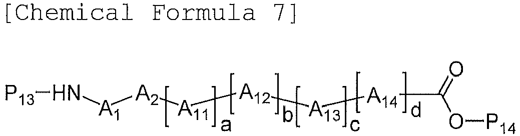 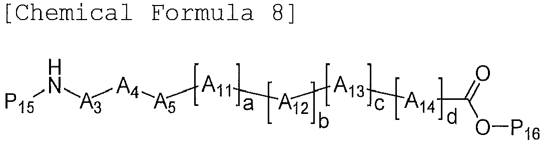 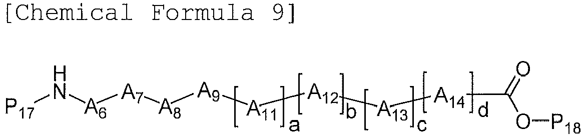 wherein
A₁ to Ag and A₁₁ to A₁₄ are independently of one another PNA monomers including a nucleic acid base, which are identical to or different from each other;
P₁ to P₁₈ are independently of one another hydrogen or protecting groups identical to or different from each other, but each of P₁ and P₂, P₃ and P₄, P₅ and P₆, P₁₁ and P₁₂, P₁₃ and P₁₄, P₁₅ and P₁₆, and P₁₇ and P₁₈ is not hydrogen at the same time; and
a and b are independently of each other an integer of 1, and c and d are independently of each other an integer of 0 or 1.

3. The method for producing a PNA oligomer of claim 2, wherein the method includes:
a) in the presence of a coupling reagent or a solvent, adding a second PNA dimer represented by the following Chemical Formula 4-1, a second PNA trimer represented by the following Chemical Formula 5-1, or a second PNA tetramer represented by the following Chemical Formula 6-1 to a first PNA dimer represented by the following Chemical Formula 1-1, a first PNA trimer represented by the following Chemical Formula 2-1, or a first PNA tetramer represented by the following Chemical Formula 3-1 to obtain a mixed solution; and
b) adding an amine compound to the mixed solution of a) to produce the compounds of Chemical Formulae 7 to 9: 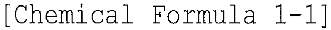 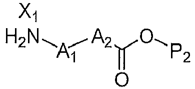 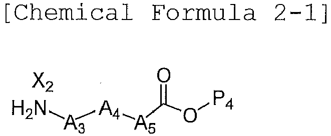 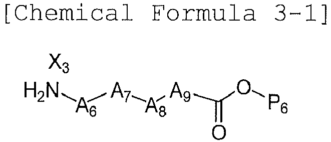 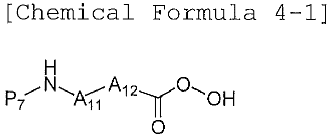 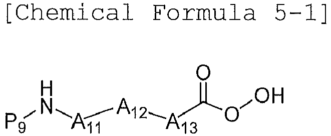 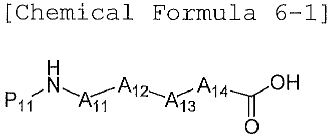 wherein
A₁ to A₉ and A₁₁ to A₁₄ are independently of one another PNA monomers including a nucleic acid base, which are identical to or different from each other;
P₂, P₄, P₆, P₇, P₉, and P₁₁ are independently of one another hydrogen or protecting groups identical to or different from each other;
X₁ to X₃ are independently of one another an acid salt; and
a and b are independently of each other an integer of 1, and c and d are independently of each other an integer of 0 or 1.

4. The method for producing a PNA oligomer of claim 2 or 3, wherein the amine compound is N,N-diisopropylethylamine.

5. The method for producing a PNA oligomer of claim 3, further comprising: using the product obtained in b) as a starting material again to repeat a) and b).

6. The method for producing a PNA oligomer of claim 3, wherein a volume ratio of the starting material and the solvent in the mixed solution is 1:10 or more.

7. The method for producing a PNA oligomer of claim 2 or 3, wherein the nucleic acid base is adenine, cytosine, 5-methylcytosine, guanine, thymine, uracil, purine, 2,6-diaminopurine, N⁴N⁴-ethanocytosine, N⁶N⁶-ethano-2,6-diaminopurine, 5-(C3-C6)-alkynyluracil, 5-(C3-C6)-alkynylcytosine, 5-(1-propargylamino)uracil, 5-(1-propargylamino)cytosine, phenoxazine, 9-aminoethoxyphenoxazine, 5-fluorouracil, pseudoisocytosine, 5-(hydroxymethyl)uracil, 5-aminouracil, pseudouracil, dihydrouracil, 5-(C1-C6)-alkyluracil, 5-(C1-C6)-alkylcytosine, 5-(C2-C6)-alkenylcytosine, 5-fluorocytosine, 5-chlorouracil, 5-chlorocytosine, 5-bromouracil, 5-bromocytosine, 7-deazaadenine, 7-deazaguanine, 8-azapurine, 7-deaza-7-substituted purine, thiouracil, or an artificial nucleic acid base.

8. The method for producing a PNA oligomer of claim 2 or 3, wherein the protecting group is fluorenylmethoxycarbonyl, tert-butyloxycarbonyl, benzyloxycarbonyl, benzhydryloxycarbonyl (Bhoc), acetyl, benzoyl, benzyl, carbamate, p-methoxybenzyl, 3,4-dimethoxybenzyl, p-methoxyphenyl, tosyl, trichloroethyl chloroformate, sulfonamides, or isobutyryl.

9. The method for producing a PNA oligomer of claim 2,
wherein the first PNA dimer or the second PNA dimer is represented by the following Chemical Formula 11,
the first PNA trimer or the second PNA trimer is represented by the following Chemical Formula 12, and
the first PNA tetramer or the second PNA tetramer is represented by the following Chemical Formula 13: 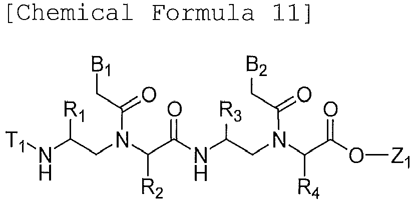 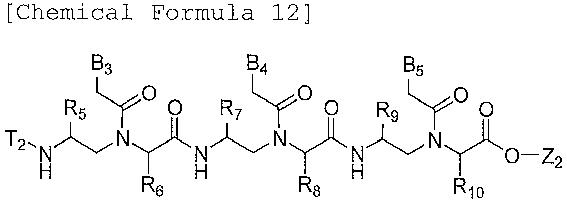 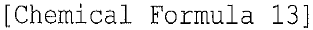 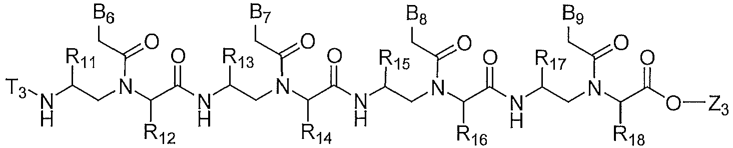 wherein
R₁ to R₁₈ are independently of one another hydrogen or an amino acid residue having a residue or substituent of an amino acid;
T₁ to T₃ are independently of one another an amine protecting group;
Z₁ to Z₃ are independently of one another a hydroxy protecting group; and
B₁ to Bg are independently of one another a nucleic acid base with or without an amine protecting group.

10. The method for producing a PNA oligomer of claim 3, wherein the first PNA dimer represented by Chemical Formula 4-1, the first PNA trimer represented by Chemical Formula 5-1, or the first PNA tetramer represented by Chemical Formula 6-1 is used at 1:0.8 to 1.2 mol with respect to 1 equivalent of the first PNA dimer represented by Chemical Formula 1-1, the first PNA trimer represented by Chemical Formula 2-1, or the first PNA tetramer represented by Chemical Formula 3-1.

11. The method for producing a PNA oligomer of claim 2 or 3, wherein the coupling reagent is HBTU, PyBop, or a mixture thereof.

12. The method for producing a PNA oligomer of claim 3, wherein b) is performed at -10 to 5°C for 10 to 60 minutes.

13. The method for producing a PNA oligomer of claim 2 or 3, wherein the solvent is a mixed solvent of chlorinated (C1-C4)alkane, DMF, and DIEA.

14. The method for producing a PNA oligomer of claim 2, further comprising: adding water to the prepared compounds of Chemical Formulae 7 to 9 to perform separation and purification.
